# EUROPEAN PATENT SPECIFICATION

(11) **EP 1 481 629 B1**
(45) Date of publication and mention of the grant of the patent: **20.02.2008**
(21) Application number: 04009963.2
(22) Date of filing: 27.04.2004
(51) Int. Cl.: A61B 1/05, A61B 1/00, A61B 1/005, G02B 23/24, G02B 7/02

(54) **Endoscope and assembly method therefor**
Endoskop und dessen Konstruktionsverfahren
Endoscope et son procédé de fabrication

(30) Priority: 30.05.2003 JP 2003155628; 10.02.2004 JP 2004033665
(43) Date of publication of application: 01.12.2004
(73) Proprietor: Olympus Corporation, Shibuya-ku, Tokyo (JP)
(72) Inventor: Matsumoto, Kazutaka, Hachioji-shi Tokyo (JP); Kaneko, Hiroyuki, Hachioji-shi Tokyo (JP); Takada, Tadatsugu, Hachioji-shi Tokyo (JP)
(74) Representative: von Hellfeld, Axel

(56) References cited:
- EP-A- 1 180 346
- US-A- 4 753 224
- US-A- 4 856 495
- US-A- 5 685 823
- US-A- 5 865 726
- US-A- 5 916 148
- US-A1- 2002 128 535
- US-A1- 2002 161 278
- US-B1- 6 361 491
- US-B1- 6 547 722

## Description

The present invention relates to an endoscope having optical members in the distal end portion of an insertion section to be inserted into the body cavity and an assembly method therefor.

In general, an illumination window portion of an illumination optical system and an observation window portion of an observation optical system are arranged in a tip member that forms the distal end portion of an insertion section of an endoscope. The illumination or observation window portion is formed by arranging various optical members, such as lenses, glass covers, etc., in an open end portion of a housing bore in the tip member. These optical members are fitted into the open end portion of the housing bore from its distal end side and fixed to the tip member by adhesive bonding.

In an endoscope described in Jpn. Pat. Appln. KOKAI Publication No. 2002-85326, an illumination lens of the illumination optical system is fitted into the open end portion of the housing bore in the tip member. A tip cover member is fitted on the tip member and serves to hold the distal-end-side peripheral edge of the illumination lens in position. The illumination lens is fixed to the tip member by adhesive bonding.

In an endoscope described in Jpn. Pat. Appln. KOKOKU Publication No. 7-4339, moreover, a housing bore is formed in a cylindrical lens frame, and an objective lens of the observation optical system is fitted into the open end portion of the housing bore from the distal end side and fixed to the lens frame by adhesive bonding. The lens frame is inserted into a bore in the tip member from the distal end side and attached to the tip member.

In the conventional endoscopes, the optical members are fitted into the open end portion of the housing bore in the tip member or the lens frame from the distal end side and fixed by adhesive bonding. According to this fixing method, it is hard accurately to position the optical members, especially with respect to the front-back direction. Thus, assembly operation for positioning the optical members is very troublesome.

United States patent no. 6,361,491 illustrates an endoscope having an optical adapter. The endoscope includes an objective lens system mounted at a distal end thereof with a mounting projection that engages a shoulder in the lens frame. This provides some improvements over other lens mounting configurations, but it remains an object to improve positioning and mounting of the optical members.

United States patent no. 4,753,224 discloses an alternative endoscope tip.

The present invention has been made in view of such problems, and its object is to provide an endoscope in which optical members in the distal end portion of an insertion section can be positioned with improved accuracy and an endoscope assembly method capable of easily assembling the endoscope of this type.

The present invention provides an endoscope and an assembly method for an endoscope having the features recited in claims 1 and 15, respectively. Preferred features of the invention are recited in the dependent claims.

According to the present invention, there is provided an endoscope according to claim 1 which has an elongate insertion section to be inserted into a body cavity with a distal end portion thereof forward. The endoscope comprises a tip member provided on the distal end portion and having a housing bore extending from a rear end side to a distal end side, a protrusion on an inner peripheral surface of the housing bore, and an optical member which is inserted into the housing bore from the rear end side and fixed to the tip member with the distal end portion thereof run against the protrusion. The optical member is inserted into the housing bore from the rear end side so that its distal end portion is run against the protrusion,
whereby the optical member is positioned.

The tip member has a distal end face, and the protrusion is located on a distal end portion of the inner peripheral surface of the housing bore and has a taper inclined to the distal end side and smoothly connected to the distal end face.

The protrusion is located on the distal end portion of the inner peripheral surface of the housing bore, and the taper, which is inclined to the distal end side and smoothly connected to the distal end face, is provided on the distal end side of the protrusion. Thus, the optical function of the optical member is prevented from being spoiled.

Preferably, the optical member includes an illumination optical member for illumination.

Thus, the illumination optical member may be inserted into the housing bore from the rear end side so that its distal end portion is run against the protrusion, whereby the illumination optical member is positioned.

Preferably, the optical member includes an observation optical member for observation.

Thus the observation optical member may be inserted into the housing bore from the rear end side so that its distal end portion is run against the protrusion, whereby the observation optical member is positioned.

Preferably, the endoscope further comprises an additional optical member which cooperates with the optical member. The inner peripheral surface of the housing bore has a distal-end-side region in which the protrusion and the optical member are arranged and a rear-end-side region which is larger in diameter than the distal-end-side region and in which at least a part of the additional optical member is located, and the tip member has a stepped portion which is formed between the distal- and rear-end-side regions and against which a distal end portion of the additional optical member is run.

Thus, the optical member is positioned in a manner such that its distal end portion is run against the protrusion, and the additional optical member may be positioned in a manner such that its distal end portion is run against the stepped portion.

Preferably, the optical member includes an illumination lens for illumination, and the additional optical member includes a light guide fiber which supplies illumination light to the illumination lens.

Thus, the illumination lens may be positioned in a manner such that its distal end portion is run against the protrusion, and the light guide fiber may be positioned in a manner such that its distal end portion is run against the stepped portion.

Preferably, the tip member includes a first member on the distal end side having the protrusion and a second member on the rear end side which holds the optical member in cooperation with the first member, between itself and the first member.

Thus, the tip member may be divided into the first and second members, and the optical member may be fixed to the tip member in a manner such that it is held between the first member on the distal end side and the second member on the rear end side.

Preferably, the first member is formed of a material capable of fill-curing.

Thus, the first member may be formed of the material capable of fill-curing, and the optical member may be positioned in a molding tool.

Preferably, the tip member has a longitudinal axis extending in a longitudinal direction of the insertion section, and the first member and the second member are block-shaped mating members individually having parting faces across a plane perpendicular to the longitudinal axis.

Thus, the first and second members may be parted into the block-shaped mating members.

Preferably, the tip member is formed having a channel extending parallel to the optical member, and the parting faces across the plane perpendicular to the longitudinal axis are located between the optical member and the channel.

Thus, the parting faces that cross the plane perpendicular to the longitudinal axis may be located between the optical member and the channel, and a distance is kept between a parting portion on the optical member side and a parting portion on the channel side.

Preferably, the tip member includes a mounting member attached to the tip member and having the housing bore.

Thus, the optical member may be inserted into the housing bore of the mounting member and positioned, and the mounting member is attached to the tip member.

Preferably, the tip member has an inner peripheral surface defining a through hole extending from the rear end side to the distal end side, an inner peripheral surface of the through hole has a first distal-end-side region and a first rear-end-side region larger in diameter than the distal-end-side region, the tip member has a first stepped portion formed between the first distal-end-side region and the first rear-end-side region, and an outer peripheral surface of the mounting member has a second distal-end-side region, a second rear-end-side region larger in diameter than the second distal-end-side region, and a second stepped portion formed between the second distal-end-side region and the second rear-end-side region and running against the first stepped portion.

Thus, the mounting member may be positioned in a manner such that its second stepped portion is run against the first stepped portion of the tip member.

Preferably, the optical member includes an objective lens for observation of which a distal end portion is run against the protrusion, and the mounting member includes a lens frame having the housing bore.

Thus, the objective lens may be inserted into the housing bore of the lens frame from the rear end side so that its distal end portion is run against the protrusion, whereby the objective lens is positioned.

Preferably, the tip member includes a first member on the distal end side having the protrusion and a second member on the rear end side which holds the mounting member in cooperation with the first member, between itself and the first member.

Thus, the tip member may be parted into the first and second members, and the mounting member may be fixed to the tip member in a manner such that it is held between the first member on the distal end side and the second member on the rear end side.

According to the present invention, there is provided an assembly method according to claim 15 for an endoscope, which has an elongate insertion section to be inserted into a body cavity with a distal end portion thereof forward. The method comprises inserting an optical member into a housing bore, which penetrates a tip member on the distal end portion from a rear end side to a distal end side, from the rear end side, positioning the optical member by running a distal end portion of the optical member against a protrusion on an inner peripheral surface of the housing bore, and fixing the optical member to the tip member.

Preferably, the positioning includes positioning the optical member by running the distal end portion of the optical member against the protrusion, in a distal-end-side region of the inner peripheral surface of the housing bore in which the protrusion is located, and positioning an additional optical member, which cooperates with the optical member, by running a distal end portion of the additional optical member against a stepped portion, which is formed between the distal-end-side region and a rear-end-side region larger in diameter than the distal-end-side region, in the rear-end-side region of the inner peripheral surface of the housing bore.

Preferably, the fixing includes holding the optical member by means of a first member on the distal end side of the tip member which has the protrusion and a second member on the rear end side of the tip member, between the first member and the second member.

The invention can be more fully understood from the following detailed description when taken in conjunction with the accompanying drawings, in which:
FIG. 1 is a side view showing an outline of an endoscope according to a preferred embodiment of the invention;
FIG. 2 is a perspective view of the distal end portion of the endoscope;
FIG. 3A is a longitudinal sectional view of a region near the distal end portion of the endoscope;
FIG. 3B is an enlarged longitudinal sectional view showing a region near the distal end of a first lens frame;
FIG. 3C is an enlarged longitudinal sectional view showing a region near the distal end of another example of a first lens frame;
FIG. 4 is a front view of the distal end portion of the endoscope;
FIG. 5 is a longitudinal sectional view of the distal end portion of the endoscope taken along line V-V of FIG. 4;
FIG. 6 is an enlarged longitudinal sectional view showing a protrusion on the distal end portion of the endoscope;
FIG. 7 is a cross-sectional view of the distal end portion of the endoscope taken along line VII-VII of FIG. 3A;
FIG. 8 is a cross-sectional view of the distal end portion of the endoscope taken along line VIII-VIII of FIG. 3A;
FIG. 9 is a cross-sectional view of the distal end portion of the endoscope taken along line IX-IX of FIG. 3A;
FIG. 10 is a cross-sectional view of the distal end portion of the endoscope taken along line X-X of FIG. 3A;
FIG. 11 is a cross-sectional view of the distal end portion of the endoscope taken along line XI-XI of FIG. 3A;
FIG. 12 is a longitudinal sectional view of a region near the distal end portion of an endoscope according to a comparative example;
FIG. 13 is a cross-sectional view of the distal end portion of the endoscope of the comparative example taken along line XIII-XIII of FIG. 12;
FIG. 14A is a cross-sectional view of the distal end portion of the endoscope of the comparative example taken along line XIVA-XIVA of FIG. 12;
FIG. 14B is a longitudinal sectional view showing the distal end portion of an observation optical system;
FIG. 15 is a longitudinal sectional view showing the distal end portion of an illumination optical system of the endoscope of the comparative example;
FIG. 16A is a longitudinal sectional view of a region near the distal end portion of an endoscope according to a further comparative example;
FIG. 16B is an enlarged longitudinal sectional view showing a region near an objective lens;
FIG. 16C is a longitudinal sectional view showing a region near the distal end of an observation optical system with first and second members disassembled;
FIG. 16D is a longitudinal sectional view showing the distal end of an illumination optical system and its surroundings with the first and second members disassembled;
FIG. 17A is a longitudinal sectional view of a region near the distal end portion of an endoscope according to a third comparative example;
FIG. 17B is an enlarged longitudinal sectional view showing a region near the distal end of an objective lens;
FIG. 18A is a longitudinal sectional view of a region near the distal end portion of an endoscope according to a fourth comparative example;
FIG. 18B is an exploded perspective view showing a region near the distal end portion of the endoscope with first and second members disassembled;
FIG. 18C is a longitudinal sectional view showing a region near the distal end of an illumination optical system;
FIG. 18D is another longitudinal sectional view of a region near the distal end portion of the endoscope, cut along a plane perpendicular to the profile of FIG. 18A;
FIG. 19 is an exploded perspective view showing a region near the distal end portion of an endoscope according to a modification of the fourth example with first and second members disassembled; and
FIG. 20 is a longitudinal sectional view of a region near the distal end portion of an endoscope according to a fifth comparative example.

An endoscope according to a preferred embodiment of the present invention will now be described with reference to the accompanying drawings. The endoscope of the present embodiment is a forward-viewing flexible endoscope for the urology that can be inserted into the urethra in use. Alternatively, the present invention may be also applied to endoscopes of various other forms, such as an endoscope for the uterus, endoscopes for lower digestive organs, etc.

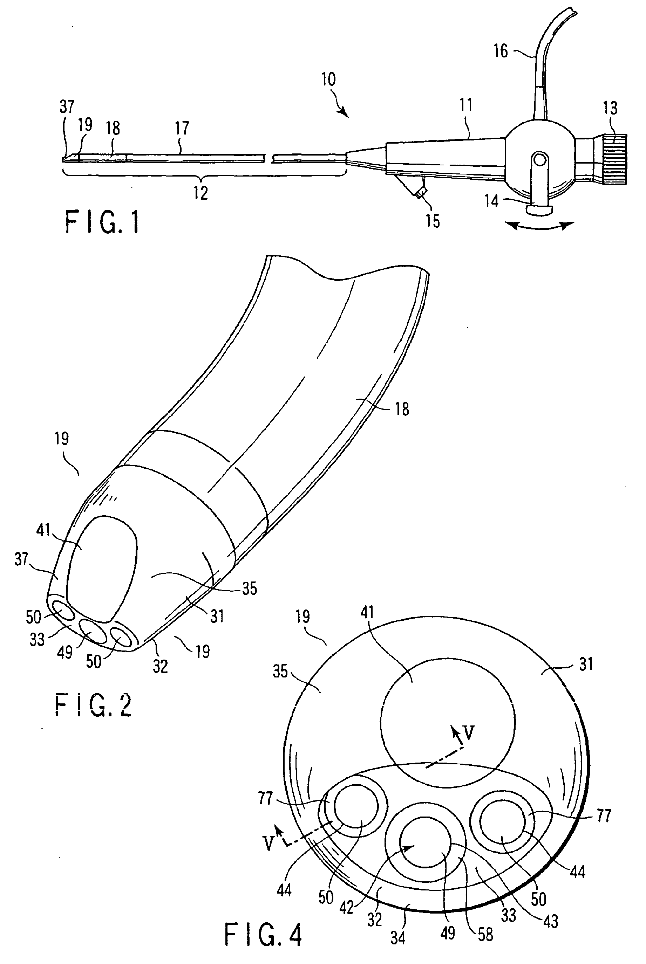

As shown in FIG. 1, an endoscope 10 according to the present embodiment has a hand control section 11 and a flexible insertion section 12. The control section 11 is provided with an eyepiece unit 13, a bending control knob 14, and a channel connector 15. Further, light guide cable 16 is joined to the control section 11. The insertion section 12 comprises a flexible tube portion 17, a bending portion 18, and a tip portion 19, which are successively joined to one another and arranged in a line from the proximal end side. The flexible tube portion 17 is a flexible portion that has resiliency and bends obeyingly when subjected to an external force with a given or higher value. Further, the bending portion 18 can be compulsorily bent in the vertical direction to reorient the tip portion 19 by turning the bending control knob 14 of the control section 11.

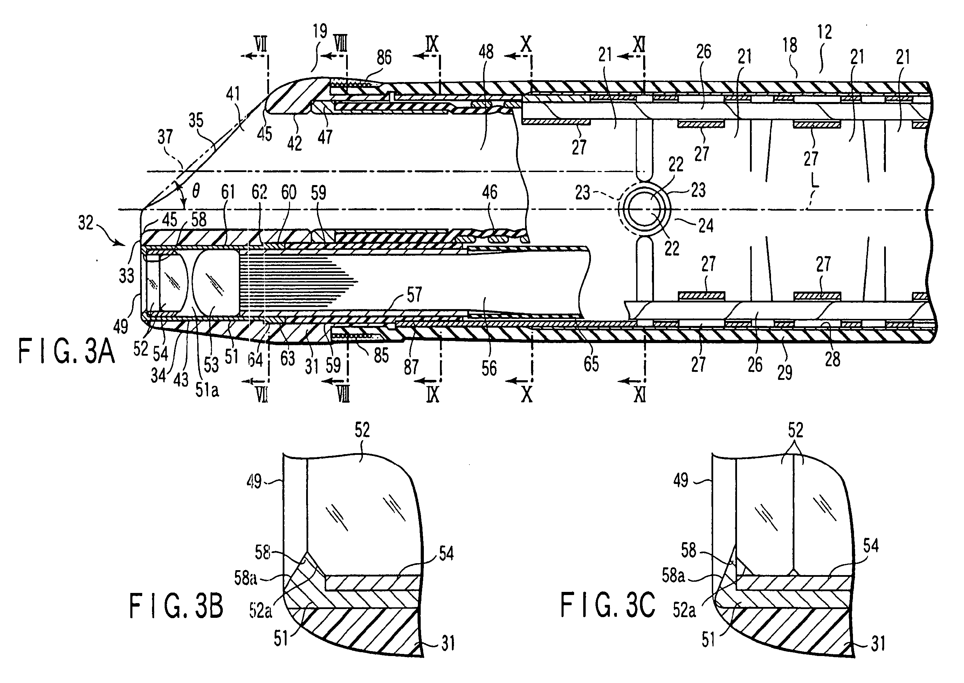

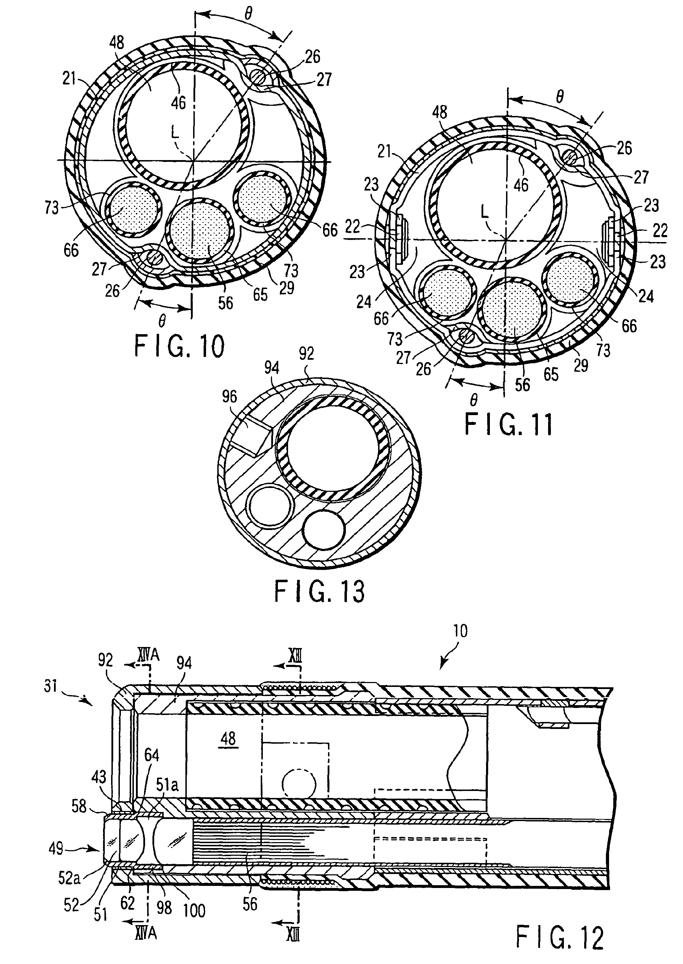

In the bending portion 18, as shown in FIG. 3A, a plurality of bending pieces 21 are arranged in a line in the direction of a central axis L (a longitudinal axis) of an insertion section 12. Each two adjacent bending pieces 21 are pivotally connected for vertical rocking motion by means of a shaft member 22. More specifically, lugs 23 are arranged individually on the left- and right-hand portions of the distal and rear end edges of each of the bending pieces 21 so as to project toward the adjacent bending piece 21, and the adjacent lugs 23 are lapped on each other. As shown in FIG. 11, moreover, each shaft member 22 is passed through both of the lapped lugs 23 and riveted, whereupon a pivot portion 24 is formed. Since the pivot portions 24 are thus formed individually on the left- and right-hand portions of the bending pieces 21, the bending portion 18 (see FIG. 1) can be bent in the upward and downward directions only. If the pivot portions 24 are arranged in the upper and lower positions as well as in the left- and right-hand positions, the bending portion 18 can be bent in the leftward and rightward directions as well as in the upward and downward directions. As shown in FIG. 3A, guide rings 27 are attached individually to substantially top and bottom portions of each bending piece 21. The upper and lower guide rings 27 are penetrated by control wires 26, individually.

As shown in FIGS. 10 and 11, the guide rings 27 and the control wires 26 that penetrate them are not accurately situated just upper and lower, and are situated with a lateral deviation for an angle θ giving priority to situation of built-in members in the tip portion 19 (see FIG. 3A). However, the bending direction is considered to be the vertical direction, and the bending function can be fulfilled without inconvenience in use.

As shown in FIG. 3A, moreover, the respective distal ends of the control wires 26 are fixed, for example, by brazing such as soldering in a manner such that they are inserted in the guide rings 27 of the leading bending piece 21. The control wires 26 are passed individually through the upper and lower guide rings 27 of the bending pieces 21. Further, they are guided into the hand control section 11 (see FIG. 1) through a guide sheath (not shown) in the flexible tube portion 17 (see FIG. 1), and coupled to a bending control mechanism (not shown) that is operated by means of the bending control knob 14 (see FIG. 1). If the knob 14 is turned, the bending control mechanism pushes and pulls the upper and lower control wires 26. The bending portion 18 can be bent upward and downward by pulling the control wires 26 to turn the bending pieces 21.

In the bending portion 18, as shown in FIG. 3A, a so-called blade 28 is put on the outer peripheries of the bending pieces 21, and its outside is covered by a sheath 29. The distal end side of the sheath 29 extends to the basal part of the tip portion 19 and covers the outer periphery of the basal part of the tip portion 19.

As shown in FIGS. 2 and 3A, the tip portion 19 of the insertion section 12 is provided with a tip forming member 31 as a tip member. The tip forming member 31 is a simple component that is molded integrally from resin. The resin as the material of the tip forming member 31 may be one that has some light transmission property. A lower part of the tip forming member 31 with respect to the bending motion forms a paddle-shaped projecting portion 32 that projects forward. As shown in FIG. 4, a distal end face 33 of the projecting portion 32 has a shape such that it is laterally long and vertically flat, e.g., the shape of an ellipse having its minor axis in the vertical direction and its major axis in the horizontal direction.

When viewed from the front, as shown in FIG. 4, the projecting portion 32 and the distal end face 33 are located substantially within the lower half of the region inside the outline of the tip forming member 31. The projecting portion 32 is situated below a slanting surface 35, which will be mentioned later. The peripheral surface of the tip forming member 31 is a smooth surface that has no sharp angles or edgy irregularities and covers the area ranging from the edge of the distal end face 33 of the projecting portion 32 to the outer periphery of the rear end portion of the tip forming member 31. More specifically, the whole area that ranges from the edge of the substantially elliptic distal end face 33 to the substantially circular outer peripheral surface of the proximal end portion of the rear half of the tip forming member 31 is formed of a continuous curved surface. The outer surface of the tip forming member 31 is a smooth curved surface that is initially substantially elliptic and then becomes substantially circular in the region that ranges from the edge of the substantially elliptic distal end face 33 to the bendable bending portion 18 with a substantially circular cross section that adjoins the rear end of the tip portion 19. As shown in FIG. 2, moreover, the projecting portion 32 projects forward from the underside of the tip forming member 31, and its distal end is tapered. A slanting surface 34 on the underside of the tip forming member 31, shown in FIG. 3A is a curved surface that is gently narrowed at a small angle to the central axis L of the insertion section 12.

As shown in FIG. 3A, on the other hand, the slanting surface 35 is formed on the surface above the projecting portion 32. The slanting surface 35 is a slope that is inclined at the relatively wide tilt angle θ to the central axis L of the insertion section 12. The slanting surface 35 is formed of a curved surface. When viewed laterally, as shown in FIG. 3A, it is a slope of which the extreme end side declines close to the central axis L of the insertion section 12. When viewed from above, moreover, the slanting surface 35 has a laterally wide shape such that its distal end side slightly narrows within the width of the basal part of the tip forming member 31, as shown in FIG. 2.

The projecting portion 32 of the tip forming member 31 and the overlying slanting surface 35 form a guide paddle portion 37. The paddle portion 37 is located in a direction such that the bending portion 18 bends or, in this case, the tip portion 19 is raised. The peripheral edge of the distal end face 33 of the projecting portion 32 and externally exposed corner portions of the tip forming member 31 are rounded.

As shown in FIG. 4, the tip forming member 31 is formed having a channel hole 42, which defines a channel opening 41, an observation bore 43, and a pair of illuminator housing bores 44, left and right. An assembly member for an observation optical system is set in the observation bore 43, and assembly members for an illumination optical system in the housing bores 44. As shown in FIG. 3A, the channel hole 42 that defines the channel opening 41 linearly extends parallel to the central axis L of the insertion section 12. When viewed from the front, as shown in FIG. 4, the channel opening 41 opens in the central part of the slanting surface 35 of the tip forming member 31.

As shown in FIGS. 1 and 3A, a channel tube 46 is connected by means of a connector 47 to the inner end of the channel hole 42 that defines the channel opening 41. The channel tube 46 is guided through the bending portion 18 and the flexible tube portion 17 to the hand control section 11 and connected to the channel connector 15. Thus, it forms a channel 48 that extends from the channel connector 15 to the channel opening 41 of the tip portion 19. The channel 48 is used for air/water feed or the like, as well as for the insertion of forceps. If air or water is fed using the channel 48, it is through an adapter of an air/water feed apparatus (not shown) that is attached to the channel connector 15. Further, the air/water feed is controlled by operating an air/water feed control button that is located on the hand control section 11. Furthermore, air or water may be fed through another air/water feed channel (not shown) that is separate from the channel 48.

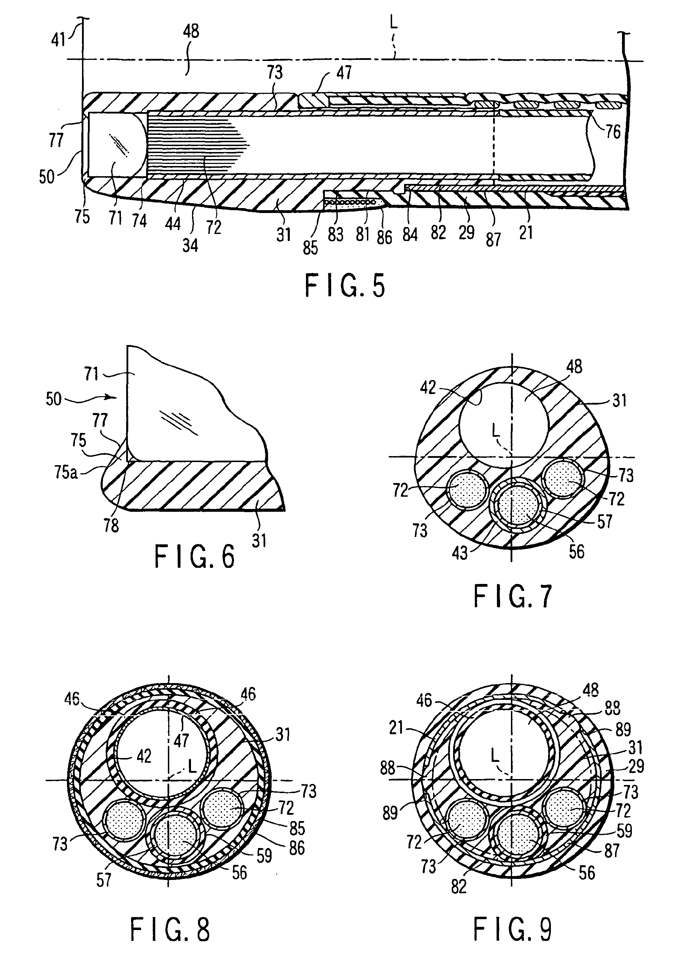

As shown in FIGS. 3A and 5, the observation bore 43, having therein the assembly member for the observation optical system, and the illuminator housing bores 44, having therein the assembly members for the illumination optical system, are formed straight in the direction of the central axis L of the insertion section 12, in the area of the projecting portion 32 of the tip forming member 31. The bores 43 and 44 penetrate the tip forming member 31. As shown in FIG. 7, the observation bore 43 is situated in the center of the projecting portion 32, while the pair of illuminator housing bores 44 are situated left and right in the projecting portion 32. Further, the bores 43 and 44 are located within the region of the distal end face 33 of the projecting portion 32. Thus, an observation window 49 and an illumination window 50 (mentioned later), which are defined by the bores 43 and 44, are also arranged within the region of the distal end face 33.

The observation optical system is constructed in the manner shown in FIG. 3A. A cylindrical first lens frame 51 as a mounting member is insertion the observation bore 43 of the tip forming member 31. A observation housing bore 51a, which is formed of the lumen of the first lens frame 51, contains first objective lens 52 as an optical member and second objective lens 53. The lenses 52 and 53 are situated on the front and rear end sides, respectively. The first and second objective lenses 52 and 53 are previously set in the first lens frame 51 before the frame 51 is mounted in the observation bore 43 of the tip forming member 31.

In this case, the first objective lens 52 is formed of a two-piece lens. It is previously assembled to a cylindrical second lens frame 54, which is inserted into the first lens frame 51 through its rear end opening and assembled to it.

Further, the second objective lens 53 is coaxially attached and fixed to a connector 57, which is attached to the distal end of an image guide fiber 56, thus forming an integrated component assembly. The second objective lens 53 and the connector 57 are substantially equal in outside diameter. Thus, the second objective lens 53, in a body with the connector 57 of the image guide fiber 56, can be inserted into the observation housing bore 51a through its rear end opening.

The second objective lens 53 and the connector 57 can move in the direction of the optical axis of the first lens frame 51 before they are fixed to the frame 51. Further, a gap is formed between the inner peripheral surface of the first lens frame 51 and the respective outer peripheral surfaces of the connector 57 and the second objective lens 53. This gap serves to make allowance for the insertion of the connector 57 and the lens 53, having an adhesive spread on their outer peripheral surfaces, into their given positions in the first lens frame 51.

The inside diameter of the first lens frame 51 is adjusted to a value such that the second lens frame 54, second objective lens 53, and the connector 57 of the image guide fiber 56, having the adhesive spread on their outer peripheral surfaces, can be inserted into their given positions. The first lens frame 51 is formed so that its inside diameter in the rear-end-side region through which components on the distal end side pass is smaller than that in the front-end-side region. The diameter on the rear end side is reduced in one or more steps to secure the positioning function for the components, while the ability of the adhesive-coated components to be inserted to the distal end side is improved.

The first lens frame 51 serves as a light shielding member that prevents leakage of light as well. Thus, it is formed of a light shielding material such as resin or metal.

As shown in FIG. 3A, a protective tube 59 is fit on the outer periphery of the connector 57 of the image guide fiber 56 so that its distal end closely abuts against the rear end of the first lens frame 51. The distal end portion of the connector 57 is inserted into the observation housing bore 51a through its rear end opening, so that the distal end of the protective tube 59 is butted against the rear end of the first lens frame 51.

As shown in FIG. 3A, a protrusion 58 in the form of an inwardly projecting claw is formed on the distal end of the inner peripheral surface which defines the observation housing bore 51a of the first lens frame 51 so as to cover the whole or part of the circumference of the inner peripheral surface. As shown in FIG. 3B, the inner end face of the protrusion 58 is engaged by a chamfered end edge portion 52a of the first objective lens 52 at the leading end that is assembled to the second lens frame 54. Alternatively, as shown in FIG. 3C, the inner end face of the protrusion 58 may be designed to be engaged by a region of the distal end face of the first objective lens 52 that is a little inside of the chamfered end edge portion 52a, as shown in FIG. 3C. Thus, when the second lens frame 54 is inserted through the rear-side opening of the observation housing bore 51a (see FIG. 3A), the chamfered end edge portion 52a or the distal end face of the first objective lens 52 at the leading end abuts against the inner end face of the protrusion 58. Thereupon, the first objective lens 52 is prevented from moving forward and is located in a given position. Thus, the protrusion 58 serves as a positioning stopper portion. Directly, it engages the chamfered end edge portion 52a of the first objective lens 52 that is assembled to the second lens frame 54, thereby locating an insertion end position for the first objective lens 52 with respect to the first lens frame 51. Indirectly, the protrusion 58 also locates an insertion end position for the second lens frame 54 with respect to the first lens frame 51. The level difference (height) of the inner end face of the protrusion 58 is adjusted to a value greater than the thickness of the distal end of the second lens frame such that the inner end face engages the distal end face of the first objective lens 52 (including the chamfered end edge portion 52a only or any other surface than the portion 52a).

Alternatively, the height of the end face inside the protrusion 58 may be adjusted to a value substantially equal to the thickness of the distal end of the second lens frame 54 such that the only the distal end of the frame 54 abuts against the inner surface of the protrusion 58. In this case, a protrusion may be formed on the distal end of the inner peripheral surface of the second lens frame 54. The protrusion have a height such that it engages the chamfered end edge portion 52a of the first objective lens 52 or the distal end face of the lens 52 (exclusive of the chamfered end edge portion 52a). Thereupon, the protrusion 58 of the observation housing bore 51a first settles the insertion end position for the second lens frame 54 with respect to the first lens frame 51, and the position of the first objective lens 52 is positioned with respect to the first lens frame 51 by means of the second lens frame 54. Thus, the first objective lens 52 and the second lens frame 54 are an optical member that is positioned by being run against the protrusion 58.

Further, the protrusion 58 is only expected to stop the forward movement of the first objective lens 52 by engaging the distal end of the lens 52. Therefore, the height of the protrusion 58 should only be substantially equal to the sum of the thickness of the second lens frame 54 and the height of the chamfered end edge portion 52a. Thus, the optical function of the first objective lens 52 cannot be spoiled if the protrusion 58 is formed on the distal end of the observation housing bore 51a.

Further, a taper 58a is formed on the distal-end-side surface of the protrusion 58. The taper 58a spreads from the observation window 49 toward the distal end, inclines radially from the center of the window 49, and substantially smoothly adjoins the distal end face of the tip forming member 31. Thus, the distal-end-side surface of the protrusion 58 has a shape such that shading of the observation field is minimized, so that the optical function can be prevented further securely from being spoiled.

As shown in FIG. 3A, on the other hand, the outer peripheral surface of a rear-end-side region 60 of the first lens frame 51 is larger in diameter than a distal-end-side region 61, and a stepped portion 62 is formed on the distal end of the rear-end-side region 60. A thick rear-end-side region 63 is formed in the observation bore 43, matching the rear-end-side region 60 and the stepped portion 62 of the first lens frame 51 in shape. A stepped portion 64 is formed on the distal end of the rear-end-side region 63. When the first lens frame 51 is inserted into the observation bore 43 from behind, as shown in FIG. 3A, its stepped portion 62 runs against the stepped portion 64 of the observation bore 43. Thereupon, an end position for the insertion of the first lens frame 51 into the observation bore 43 is settled. Thus, the first lens frame 51 can be inserted only from behind the observation bore 43, and the stepped portion 62 constitutes a stopper that regulates the end position for the insertion of the frame 51 into the observation bore 43. The first lens frame 51 is fixed to the tip forming member 31 with an adhesive or the like in this regulated position for fitting. The inside diameter of the observation bore 43 and the outside diameter of the first lens frame 51 are adjusted to values that allow the frame 51 having the adhesive spread on its outer peripheral surface to be inserted into a given position in the observation bore 43 of the tip forming member 31.

As shown in FIG. 3A, a flexible tube 65 for protection is fit on that part of the image guide fiber 56 which extends rearward from the connector 57. The image guide fiber 56 is guided to the hand control section 11 through the bending portion 18 and the flexible tube portion 17 and connected to the eyepiece unit 13.

The following is a description of a method of assembling the first and second objective lenses 52 and 53, image guide fiber 56, etc. in the observation bore 43 of the tip forming member 31.
(1) The first objective lens 52 is assembled to the second lens frame 54 that is not assembled to the first lens frame 51 yet, thereby forming an integrated component assembly (assembly process for first objective lens component assembly).
(2) Thereafter, the component assembly of the first objective lens 52, the second objective lens 53, and the connector 57 of the image guide fiber 56 to which the second objective lens 53 is fixed in advance are successively inserted into and assembled to the observation housing bore 51a of the first lens frame 51 that is not assembled to tip forming member 31 yet, from behind (assembly process for first lens frame component assembly).
   (2-1) In an assembly process for the component assembly of the first lens frame 51, the adhesive is first applied to the outer periphery of the second lens frame 54, and the frame 54 is inserted through the rear end opening of the observation housing bore 51a and pushed deep into the bore 51a by means of a tool (not shown) (insertion process for first objective lens). Thereupon, the chamfered end edge portion 52a of the first objective lens 52 runs against the protrusion 58 of the housing bore 51a, and the lens 52 stays there without being allowed to move further forward (positioning process for first objective lens). In this position, the adhesive is hardened, and the second lens frame 54 is fixed (fixing process for first objective lens).
   (2-2) Then, the adhesive is applied to the respective outer peripheries of the second objective lens 53 and the connector 57, and the lens 53 and the connector 57 are inserted through the rear end opening of the observation housing bore 51a (insertion process for second objective lens). When the second objective lens 53 approaches the first objective lens 52, the eyepiece unit 13 or a separately prepared tool is looked in as the depth of insertion of the connector 57 is adjusted. The adjustment is stopped when the focus is obtained. The adhesive is left and hardened in this state, whereupon the second objective lens 53 and the connector 57 are fixed to the first lens frame 51 (fixing process for second objective lens component assembly).

   By doing this, the first and second objective lenses 52 and 53, second lens frame 54, and connector 57 of the image guide fiber 56 can be unitized with the first lens frame 51, and the optical adjustment can be finished before they are assembled to the tip forming member 31 of the endoscope 10.
(3) Thereafter, the component assembly of the first lens frame 51 is attached to the tip forming member 31 (attachment process for first lens frame).

In this process, the adhesive is applied to the outer periphery of the first lens frame 51, and the frame 51 is inserted into the observation bore 43 of the tip forming member 31 of the endoscope 10 through its rear opening (inserted in process for first lens frame). Thereupon, as shown in FIG. 3A, the stepped portion 62 of the first lens frame 51 runs against the stepped portion 64 of the observation bore 43, and the attachment position of the first lens frame 51 is settled (positioning process for first lens frame). In this position, the adhesive is hardened, and the first lens frame 51 is fixed to the tip forming member 31 (fixing process for first lens frame).

In this manner, the first and second objective lenses 52 and 53 and the image guide fiber 56 are attached to the first lens frame 51 and preassembled as a component assembly, and the optical adjustment is finished in advance for them. Thus, the optical adjustment requires only simple operation. Accordingly, the optical adjustment operation is easier than in the case where components are optically adjusted while they are assembled to the body of the endoscope 10. Besides, other or peripheral components can be prevented from being spoiled together by any mistake in the optical adjustment, so that lowering of the yield of product can be prevented, and the productivity can be improved. Further; the component assembly having previously undergone the optical adjustment is inserted into the observation bore 43 of the tip forming member 31 from behind. Thus, positioning and assembling operations for the component assembly are easy, so that the assembly performance is also improved, and the number of essential components can be reduced.

Furthermore, the second lens frame 54 is inserted into the observation housing bore 51a of the first lens frame 51 through its rear opening, and is run against the protrusion 58 of the housing bore 51a from inside. Therefore, the frame 54 can be easily positioned with respect to the direction of the optical axis, and it can be securely fixed in the given position. Since the protrusion 58 is integrally formed on the first lens frame 51, moreover, the former can be easily fabricated together with the latter even it is fine and small-sized. If the first lens frame 51 is made of resin, it can be integrally formed by molding with particular ease.

In assembling the first lens frame 51 to the tip forming member 31, furthermore, the frame 51 is inserted into the observation bore 43 of the member 31 through the rear opening, and the stepped portion 62 of the first lens frame 51 is positioned by being run against the stepped portion 64 of the observation bore 43. Therefore, positioning the first lens frame 51 is easy, and the frame 51 can be securely fixed in the given position in the observation bore 43 of the tip forming member 31.

The illumination optical system is constructed in the manner shown in FIG. 5. An illumination lens 71 as an optical member is directly set in each illuminator housing bore 44 of the tip forming member 31. The illumination lens 71 is situated in the leading end region of the bore 44 and fixed in the position by adhesive bonding. A cylindrical tip connector 73, which is mounted on the outer periphery of the distal end portion of a light guide fiber 72 as another optical member, is set in the rear end side of the illumination lens 71 and fixed to the tip forming member 31 also by adhesive bonding. Both the illumination lens 71 and the tip connector 73 are inserted into the illuminator housing bore 44 from its rear opening and are fixed in their respective setting positions by adhesive bonding.

The lens 71 and the connector 73 have their respective outside diameters such that there is a margin for the adhesive between the inner surface of the illuminator housing bore 44 and their outer peripheries. Therefore, the adhesive is not removed yet when the illumination lens 71 and the tip connector 73 are inserted into the given setting positions. Further, the inside diameter of the rear part of the bore 44 in which the tip connector 73 is set is a little larger than that of the part in which the illumination lens 71 is set. Thus, a stepped portion 74 against which the connector 73 is run is formed between these two parts. An end position for the insertion of the connector 73 can be regulated by running the distal end of the connector 73 against the stepped portion 74.

The inside diameter of the distal-end-side region may be made larger than the outside diameter of the tip connector 73 of the light guide fiber 72 so that the connector 73 can go forward beyond the stepped portion 74.

As shown in FIG. 5, moreover, an inward protrusion 75 is integrally formed on the distal edge of the inner peripheral surface which defines the illuminator housing bore 44 of the tip forming member 31. The protrusion 75 is formed covering the whole or part of the circumference of the inner peripheral surface. The protrusion 75 serves as a positioning stopper portion. It locates the setting position of the illumination lens 71 with respect to the illuminator housing bore 44 of the tip forming member 31 in a manner such that the distal end of the lens 71 finally runs against the inner end of the protrusion 75 when the lens 71 is inserted into the bore 44 through its rear side opening.

As shown in FIG. 6, the height of projection of the protrusion 75 must only be substantially equal to the height of a chamfered portion 78 of the edge of the illumination lens 71. In this case, the protrusion 75 is a little higher than the chamfered portion 78. The projection of the protrusion 75 is small. Further, a stepped end face is formed on the rear end side of the protrusion 75. It is a little higher than the chamfered portion 78 on the peripheral edge of the distal end of the illumination lens 71. Further, a taper 75a is formed on the distal end side of the protrusion 75. It spreads from the illumination window 50 toward the distal end, inclines radially from the center of the window 50, and substantially smoothly adjoins the distal end face of the tip forming member 31. Thus, the distal end side of the protrusion 75 has a shape such that shading of the observation field is minimized, so that the optical function cannot be spoiled.

The protrusion 75 is a fine, small-sized portion that has the stepped end face on the rear end side and a slanting surface 77 on the distal end side. The protrusion 75 can be manufactured with ease, since it is molded together with the tip forming member 31.

As shown in FIG. 5, moreover, a flexible tube 76 for protection is fit on the outer periphery of the light guide fiber 72 that extends rearward from the rear end of the tip forming member 31. In this state, the light guide fiber 72 is guided to the hand control section 11 through the bending portion 18 and the flexible tube portion 17 shown in FIG. 1, and moreover, to the light guide cable 16. In operating the endoscope 10, a connector of the light guide cable 16 is connected to an endoscopic light source unit (not shown) .

The following is a description of a method of fixing the illumination lens 71 and the light guide fiber 72 in the illuminator housing bore 44 of the tip forming member 31.
(1) First, the illumination lens 71 is assembled to the illuminator housing bore 44 of the tip forming member 31.
   In this case, the adhesive is applied to the periphery of the illumination lens 71, and the lens 71 is inserted into the illuminator housing bore 44 of the tip forming member 31 through its rear opening and pushed deep into the bore 44 by means of an insertion tool (not shown) (insertion process for illumination lens). Since that part of the bore 44 which is situated behind the stepped portion 74 has a relatively large inside diameter, as mentioned before, the lens 71 can be easily inserted into the setting position. Further, the adhesive that is spread around the illumination lens 71 cannot be scraped off as the lens 71 is inserted.
   If the illumination lens 71 is pushed into the illuminator housing bore 44, the peripheral edge of the distal end of the lens 71 finally runs against the rear end face of the protrusion 75 to be positioned thereby (positioning process for illumination lens). Thus, the lens 71 that is pushed into the bore 44 finally engages the rear end face of the protrusion 75, so that it can be securely prevented from further moving forward and positioned accurately. When the illumination lens 71 is thus positioned by means of the protrusion 75, the adhesive can be hardened securely to fix the lens 71 to the tip forming member 31 (fixing process for illumination lens).
   This process of adhesive hardening may be carried out simultaneously with the process of hardening the adhesive on the tip connector 73 of the light guide fiber 72 after the positioning process for the fiber 72, which will be mentioned later.
(2) Then, the light guide fiber 72 is assembled to the illuminator housing bore 44 of the tip forming member 31.

After the illumination lens 71 is assembled in this manner, the light guide fiber 72 may be assembled to the illuminator housing bore 44. In doing this, the adhesive is previously applied to the outer periphery of the tip connector 73 of the fiber 72, and the connector 73 is inserted into the bore 44 through its rear opening (insertion process for light guide fiber). As the tip connector 73 is pushed into the illuminator housing bore 44, its distal end finally runs against the stepped portion 74, whereupon the distal end position of the connector 73 can be settled (positioning process for light guide fiber). In this position, the adhesive is hardened, and the tip connector 73 is fixed (fixing position for light guide fiber).

Means for assembling the lens and the light guide fiber to the bore of the body member can be also utilized for assembling the objective lenses and the image guide fiber directly in the observation housing bore.

In this manner, the illumination lens 71 is inserted into the illuminator housing bore 44 from behind and securely positioned by means of the protrusion 75. Thus, the lens 71 can be accurately positioned with respect to the front-back direction and fixed securely. Further, the assembly performance of the illumination lens 71 and the light guide fiber 72 can be improved.

The following is a description of an assembling structure for coupling the bending portion 18 and the tip portion 19 of the insertion section 12. As shown in FIG. 5, first and second fitting portions 81 and 82 are formed on the outer periphery of the rear end portion of the tip forming member 31. These fitting portions, which are smaller in diameter than the distal end side, are situated in front and in the rear. The first fitting portion 81 is located on the distal end side of the second fitting portion 82, and the former is a little larger in outside diameter than the latter. A shallow circumferential groove 83 is formed on the outer peripheral surface of the first fitting portion 81. As shown in FIG. 5, the distal end portion of the sheath 29 that extends from the bending portion 18 is fit on the first fitting portion 81, and a thread 85 is wound around the distal end portion of the sheath 29 and stiffened with an adhesive 86.

As shown in FIG. 5, moreover, a cylindrical distal end portion 87 of the leading bending piece 21 of the bending portion 18 is fitted tight on the outer periphery of the second fitting portion 82. The distal end portion of the leading bending piece 21 runs against a stepped portion 84 that is formed between the first and second fitting portions 81 and 82 of the tip forming member 31, and is positioned without being pushed in further.

As shown in FIG. 9, moreover, one or more retaining protuberances 88 are formed on the outer periphery of the second fitting portion 82. The retaining protuberances 88 are formed integrally with the tip forming member 31 by molding. The protuberances 88 are fitted individually in holes 89 that are formed in the cylindrical distal end portion 87 of the leading bending piece 21 in which the second fitting portion 82 of the tip forming member 31 is fitted. Thus, the protuberances 88 can be engaged only in given positions. As shown in FIG. 9, moreover, each retaining protuberance 88 is raised like a hump, and its height should preferably be smaller than or substantially equal to the depth of each hole 89. Possibly, the height of each protuberance 88 may be made greater than the depth of each hole 89. In this case, however, the protuberances 88 should preferably be low enough not to push up the sheath 29. Naturally, the protuberances 88 may be so high that they push up the sheath 29.

In the present embodiment, the number of sets of the retaining protuberances 88 and their corresponding holes 89 is two. If three or more sets are provided, the protuberances are arranged asymmetrically with respect to the central point of the tip forming member 31. According to the present embodiment, the protuberances 88 are located in two asymmetric positions, a side position and an upper right position, as shown in FIG. 9. Thus, each retaining protuberance 88 engages only its corresponding hole 89 in its specific position only, so that the position in which the tip forming member 31 is assembled to the distal end portion 87 of the bending piece 21 is settled.

Thereupon, the tip forming member 31 of the tip portion 19 is assembled to the leading bending piece 21 in the following manner. First, the second fitting portion 82 of the tip forming member 31 is inserted into the distal end portion 87 of the bending piece 21 through its front end opening, as shown in FIG. 5. The member 31 is inserted so that the distal end of the bending piece 21 runs against the stepped portion 84. Thus, the tip forming member 31 is positioned with respect to the front-back direction. When this is done, the retaining protuberances 88 of the tip forming member 31 must be pushed into the cylindrical distal end portion 87 of the bending piece 21. The tip forming member 31 is made of resin, the height of each protuberance 88 is substantially equal to the thickness of the distal end portion 87 of the bending piece 21, and each protuberance 88 is raised like a hump. Therefore, the region to which the retaining protuberances 88 are attached can be smoothly pushed into the distal end portion 87 of the bending piece 21 while allowing the protuberances 88 to be slightly deformed. In order to facilitate the retaining protuberances 88 to escape in this case, the rear end portion of the tip forming member 31 to which the protuberances 88 are attached is formed having the shape of a cylinder, as indicated by two-dot chain line in FIG. 9, or the regions to which the protuberances 88 are attached are formed like strips that are easily deformed inward. Thus, the insertion becomes easier. The former configuration is preferably used to secure retention force.

Further, the positional relationship is contemplated as the second fitting portion 82 of the tip forming member 31 is pushed into the distal end portion 87 of the bending piece 21. Although the retaining protuberances 88 may possibly be able to get into the holes 89 in the bending piece 21 at a time, therefore, they cannot be fitted in the holes 89 in case of misalignment around the axis. In this case, the bending piece 21 or the tip forming member 31 is relatively turned around the central axis L so that the retaining protuberances 88 are fitted individually in the holes 89. In other words, the positional relationship with which the protuberances 88 and the holes 89 engage one another is explored. If they are aligned, the protuberances 88 and holes 89 engage automatically, whereupon the engagement is finished. An operator can easily recognize this engagement by a clicking feeling or sound that is produced when the protuberances 88 fall into the holes 89. Once the retaining protuberances 88 engage the holes 89, the given position for the engagement is secured, so that the subsequent processes of operation can be carried out with ease. Positioning for the assembly is simple and easy. Despite the construction that requires only a small number of components, moreover, the assembly in the given position can be accomplished with improved accuracy.

Thereafter, the distal end portion 87 of the bending piece 21 is adhesively bonded and fixed to the second fitting portion 82 of the tip forming member 31. This fixing process may be omitted, or otherwise, they may be fixed by using an alternative means.

Subsequently, the distal end portion of the sheath 29 that extends from the bending portion 18 is put on the second fitting portion 82, and the thread 85 is wound around the distal end portion of the sheath 29 and stiffened with the adhesive 86.

In the present embodiment described above, the lenses are used as the optical members. Alternatively, however, glass covers, filters, etc. may be used as the optical members. Further, glass, plastics, etc., which can transmit light, may be used as their material.

A comparative example will now be described with reference to FIGS. 12 to 15. Like reference numerals are used to designate like portions of the preferred embodiment and the comparative example that have the same functions, and a description of those portions is omitted. In the present example, a tip forming member 31 is parted in two parts, first and second members 92 and 94 in front and in the rear, and a first lens frame 51 and the like are sandwiched between the members 92 and 94. Further, the tip forming member 31 is a cylindrical member that is not provided with the projecting portion 32, slanting surfaces 34 and 35, etc. (see FIG. 3A).

As shown in FIG. 12, the tip forming member 31 comprises the front-side first member 92, which has a first objective lens 52 and the like therein, and the rear-side second member 94, which has an image guide fiber 56 and the like therein. The first and second members 92 and 94 are bonded together and fixed to each other by means of a screw 96, as shown in FIG. 13. Alternatively, the first and second members 92 and 94 may be fixed by laser welding.

Referring again to FIG. 12, the inside diameter of an observation bore 43 of the first member 92 is smaller than that of an observation bore 43 of the second member 94. Therefore, a stepped portion 64 is formed between the first and second members 92 and 94. A stepped portion 62 of the first lens frame 51 is run against the stepped portion 64.

A flange portion 98 is formed behind the stepped portion 62 of the first lens frame 51. The flange portion 98 is fitted in a spot facing portion 100 in the observation bore 43 of the second member 94. As this is done, the first objective lens 52 of the first lens frame 51 is optically positioned with respect to the second member 94.

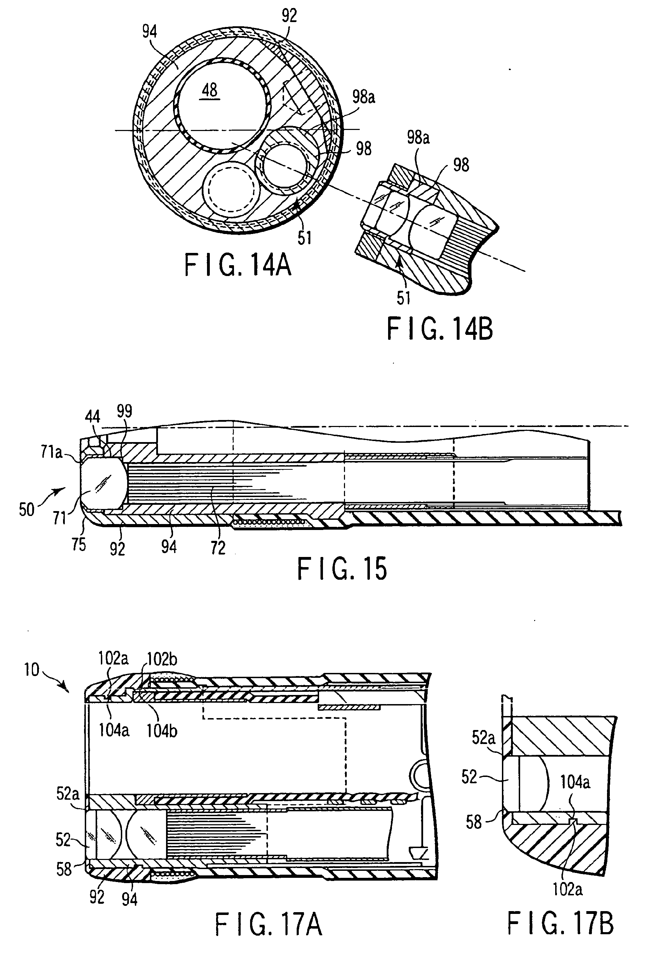

As shown in FIGS. 14A and 14B, the cross section of the flange portion 98 that is perpendicular to the longitudinal direction of insertion section has a tongue piece 98a that projects in one direction. The tongue piece 98a is located so as not to interfere with an illumination optical system, channel 48, etc. The tongue piece 98a is entirely held between the first and second members 92 and 94. Thus, the flange portion 98 is held between the first and second members 92 and 94, and the first lens frame 51 is fixed by means of the members 92 and 94. The second lens frame 54 (see FIGS. 3A to 3C) is not used in the present example.

As shown in FIG. 15, an illumination lens 71 and a light guide fiber 72 are located in the first and second members 92 and 94, respectively. The illumination lens 71, like the first objective lens 52 (see FIG. 12), is formed having a chamfer 71a on its front end face. The chamfer 71a is run against a protrusion 75 at the front end of an illuminator housing bore 44. In an illuminator housing bore 44 of the second member 94, on the other hand, its distal-end-side region is larger in inside diameter than its rear-end-side region. Therefore, a stepped portion 99 is formed between the front- and rear-end-side regions. The rear end of the lens 71 is run against the stepped portion 99. Thus, the illumination lens 71 is held and fixed between the first and second members 92 and 94.

As shown in FIGS. 12 and 15, the parting portion between the first and second members 92 and 94 is located in a central region between the first lens frame 51 and the illumination lens 71. Joints between the first lens frame 51 and the illumination lens 71 and the first and second members 92 and 94 ensure the same bond length in the longitudinal direction of the insertion section as in conventional endoscopes. Thus, the watertight function of the parting portion between the first and second members 92 and 94 can be secured.

Referring to FIGS. 12 and 15, an endoscope 10 according to the present example is supposed to be very thin (for example, having an outside diameter of 5 mm or less) and to require a function for the treatment of the affected region as well as the observation function. More specifically, the first objective lens 52, illumination lens 71, channel 48, etc. must be arranged in the thin endoscope 10. In view of spatial restrictions, therefore, the first objective lens 52 and the illumination lens 71 are expected to have very small diameters (for example, about 0.6 mm).

The first objective lens 52 and the illumination lens 71 can be run against the protrusions 58 and 75, respectively, only if the minimum inside diameter of each protrusion is smaller than the outside diameter of each corresponding lens. In some cases, the first objective lens 52 may fail satisfactorily to engage the protrusion 58, owing to misalignments of the position of the observation housing bore 51a and the combination of the first lens frame 51 and the first objective lens 52 and the like, which are attributable to variation in component tolerance. Owing to misalignments of the position of the illuminator housing bore 44 and the combination of the first and second members 92 and 94 and the like, moreover, the illumination lens 71 may possibly fail satisfactorily to engage the protrusion 75. Accordingly, the protrusions 58 and 75 should be formed having largish sizes. If the protrusions 58 and 75 are large-sized, however, an observation window 49 and an illumination window 50 are inevitably narrowed. If the first objective lens 52 and the illumination lens 71 have small diameters, in particular, the optical performance is lowered considerably. From this viewpoint, the protrusions 58 and 75 must be made smallish.

In the present example, the dimensional accuracies and geometric tolerances in the opening positions and diameters of the components and the thickness and height of the protrusions 58 and 75 are regulated highly severely, so that the protrusions 58 and 75 are allowed satisfactorily to be engaged by the first objective lens 52 and the illumination lens 71, and the optical performance of the first objective lens 52 and the illumination lens 71 cannot be spoiled. Furthermore, the protrusions 58 and 75 are worked with very strict dimensions, a thickness of 0.05 mm and height of 0.05 mm.

Preferably, on the other hand, the respective chamfers 52a and 71a of the first objective lens 52 and the illumination lens 71 should be minimized in size to maintain the optical performance. Depending on the shapes of the chamfers 52a and 71a, moreover, the lenses 52 and 71 sometimes may be recessed or projected from the distal end face of the first member 92. If they are recessed too deep, the problem of shading arises. If the lenses are projected too much, external light is transmitted through the projected surface to be incident, thereby arousing the problem of flares. Thus, the dimensions of the chamfers 52a and 71a of the lenses 52 and 71 are limited to a possible minimum value, 0.1 mm, within a range in which a precision work is practicable for workability.

For the individual components, the geometric tolerances of relevant dimensions that can cause optical axis misalignment as the components are assembled to one another with reference to the opening positions are regulated severely. The components are fitted with one another in a manner such that they can be assembled together with the first objective lens 52 as a reference. Further, the illumination lens 71 is designed with a clearance that can allow dislocation for the component tolerance.

In order to realize this processing operation, the first member 92 is formed of a metallic material or the like that is capable of fine processing. Generally, it is hard to carry out this processing operation for a resin part that covers the distal end of the endoscope 10.

The following is a description of assembly processes for the endoscope 10 according to the present example constructed in this manner. In fixing the first lens frame 51, the flange portion 98 of the frame 51 is fitted into the spot facing portion 100 of the observation bore 43 of the second member 94. Then, the flange portion 98 is held and fixed between the first and second members 92 and 94. When this is done, the first lens frame 51 is adhesively bonded to the members 92 and 94. Likewise, the rear end side of the illumination lens 71 is fitted into the distal-end-side region of the observation bore 43. Then, the lens 71 is held and fixed between the protrusion 75 of the first member 92 and the stepped portion 74 of the illuminator housing bore 44 of the second member 94. When this is done, the illumination lens 71 is adhesively bonded to the first and second members 92 and 94.

The above configuration produces the following effects. The tip forming member 31 is parted in two parts, the first and second members 92 and 94 in front and in the rear. The first lens frame 51 and the illumination lens 71 are held and fixed by means of these members 92 and 94 as well as by the adhesive fixing, between these members 92 and 94. Thus, the frame 51 and the lens 17 are fixed firmly.

Further, screws or any other mounting members, cover members, etc. are not used to fix the first lens frame 51 and the illumination lens 71 firmly to the tip forming member 31. More specifically, there are used none of screws or other mounting members that increase the outside diameter of the tip forming member 31 and cover members that increase the length of the member 31. Thus, the first lens frame 51 and the illumination lens 71 can be fixed firmly to each other without increasing the outside diameter or length of the tip forming member 31 of the endoscope 10.

The parting portion between the first and second members 92 and 94 is located in the central region between the first lens frame 51 and the illumination lens 71. The joints between the first lens frame 51 and the illumination lens 71 and the first and second members 92 and 94 ensure the satisfactory bond length in the longitudinal direction of the insertion section. Thus, the watertight function of the parting portion between the first and second members 92 and 94 can be secured.

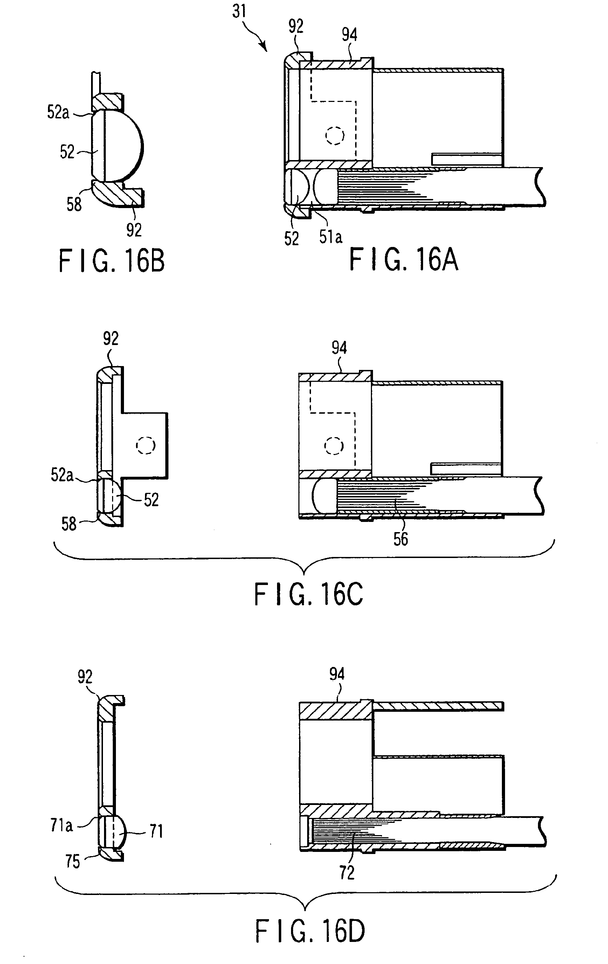

FIGS. 16A to 16D show a further comparative example. Like reference numerals are used to designate like portions of the above embodiment and example that have the same functions, and a description of those portions is omitted. In the present example, as shown in FIG. 16A, the first lens frame 51 (see FIG. 12) is not used, and a observation housing bore 51a is formed in a tip forming member 31. Thus, a first objective lens 52 is directly attached to a first member 92, as shown in FIG. 16B.

The following is a description of assembly processes for an endoscope 10 according to the present example constructed in this manner. In the present example, as shown in FIGS. 16C and 16D, the first objective lens 52 and an illumination lens 71 are first fixed to the first member 92. As this is done, the lenses 52 and 71 are fixed and assembled together by adhesive bonding in a manner such that their respective end faces are substantially flush with the end face of the first member 92. The adhesive that is oozed out onto the end faces of the lenses 52 and 71 is wiped away in advance. Thereafter, the first member 92 is fitted and fixed on a second member 94.

The above configuration produces the following effects. The first objective lens 52 and the illumination lens 71 are fixed to the first member 92, and the first member 92 is then fixed to the second member 94. Therefore, the lenses 52 and 71 are influenced little by the combinations of the other components as they are fixed to the first member 92. Thus, the very severe restrictions on the dimensional accuracies according to the preferred embodiment can be eased. The relevant dimensions include the dimensions of the chamfers 52a and 71a and the protrusions 58 and 75, the dimensions of the first and second members 92 and 94 for location adjustment, etc.

Since the first objective lens 52 and the illumination lens 71 are influenced little by the combinations of the other components as they are fixed to the first member 92, moreover, they can be arranged so that their respective end faces are substantially flush with the end face of the first member 92. Thus, undesirable optical phenomena, such as shading, flares, etc., can be prevented satisfactorily.

Since the respective end faces of the first objective lens 52 and the illumination lens 71 are substantially flush with the end face of the first member 92, the oozed adhesive can be easily wiped away when the lenses 52 and 71 are adhesively bonded to the first member 92. Further, odds of the adhesive can be removed thoroughly with ease, and the fill of the adhesive can be readily recognized. Thus, the assembly performance and bond quality are improved.

Furthermore, the first member 92 shares the function with the first lens frame 51 of the comparative example. Thus, the first lens frame 51 can be omitted, so that the endoscope 10 can be made thinner.

FIGS. 17A and 17B show a third comparative example. Like reference numerals are used to designate like portions that have the same functions, and a description of those portions is omitted. Referring to FIG. 17A, a first member 92 of the present example is formed by loading a molding tool with a plastic material by injection. The first objective lens 52 and the illumination lens 71 (see FIG. 15) are positioned in the molding tool. Thus, a watertight function is secured between the first member 92 and the lenses 52 and 71. The first member 92 may be formed of any material such as resin or rubber that cures after loading.

Further, a plurality of projections 102a and 102b are formed extending in the circumferential direction on the inner peripheral surface of the first member 92. A plurality of recesses 104a and 104b are formed extending in the circumferential direction in the outer peripheral surface of a second member 94. The recesses 104a and 104b engage with the projections 102a and 102b, respectively. Thus, the first and second members 92 and 94 are fixed to each other.

The following is a description of assembly processes for an endoscope 10 according to the present example constructed in this manner. The first member 92 is formed by loading a molding tool with a plastic material by injection. In doing this, the first objective lens 52 and the illumination lens 71 are positioned in the molding tool. The projections 102a and 102b of the first member 92 are caused to engage the recesses 104a and 104b of the second member 94, respectively, and the two members 92 and 94 are fixed to each other.

The above configuration produces the following effects. The first member 92 is formed by loading the molding tool with the plastic material by injection. The first objective lens 52 and the illumination lens 71 are positioned in the molding tool. When the lenses 52 and 71 are assembled, therefore, they are hardly influenced by the dimensions of the chamfers 52a and 71a and the protrusions 58 and 75, the dimensions of the first and second members 92 and 94 for location adjustment, etc. Thus, the respective end faces of the first objective lens 52 and the illumination lens 71 can be aligning more accurately with the end face of the first member 92.

Further, the use of the adhesive can be eliminated from the process of assembling the first objective lens 52 and the illumination lens 71 to the first member 92. Accordingly, there is no need of wiping away the adhesive that is oozed out onto the end faces of the lenses 52 and 71 or of recognizing the fill of the adhesive. Thus, the assembly performance is improved.

Since the first objective lens 52 and the illumination lens 71 are positioned in the molding tool, moreover, the watertight function can be secured between the lenses 52 and 71 and the first member 92 when the first member 92 is molded. Accordingly, there is no need of using any filling material to secure the watertight function after the molding. Thus, the assembly performance is further improved.

If the first member 92 is formed of rubber, improvement of the watertight function can be expected.

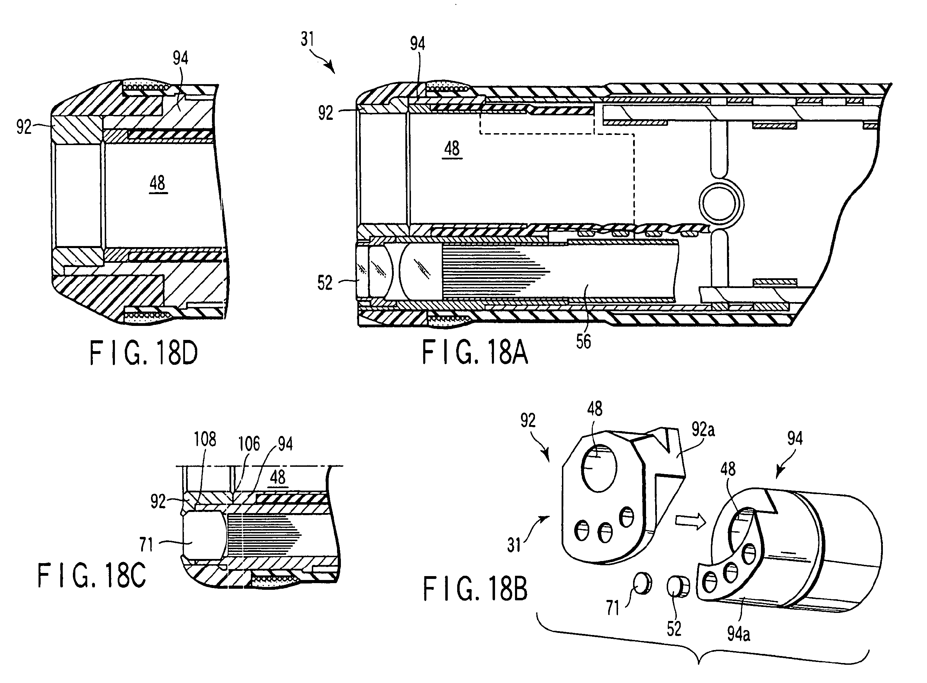

FIGS. 18A to 18D show a fourth comparative example. Like reference numerals are used to designate like portions that have the same functions, and a description of those portions is omitted. In the present example, a tip forming member 31 is parted into first and second block-shaped members 92 and 94 that mate with each other.

As shown in FIG. 18A, the tip forming member 31 of the present example has an irregular shape that is not cylindrical. The first and second members 92 and 94 are in the form of a block each. More specifically, as shown in FIG. 18B, the second member 94 has a prominence 94a on the side of an optical system that includes an image guide fiber 56 (see FIG. 18A), etc. The first member 92 has a prominence 92a on the side of a channel 48, and is shaped so that it can be fitted on the second member 94, as indicated by the arrow in FIG. 18B. The respective opposite surfaces of the prominences 92a and 94a of the first and second members 92 and 94 cross a plane that is perpendicular to the longitudinal axis of the tip forming member 31, and are located between the optical system and the channel 48. As shown in FIG. 18C, therefore, an adequate distance is kept between a parting portion 106 between the members 92 and 94, which faces the channel 48, and a parting portion 108 that faces the optical system.

The above configuration produces the following effects. The first and second members 92 and 94 are block-shaped mating members individually having parting faces that cross a plane perpendicular to the longitudinal axis of the tip forming member 31. If the tip forming member 31 has an irregular shape, not cylindrical, therefore, it can be appropriately parted into the first and second members 92 and 94.

Further, the adequate distance is kept between the parting portion 106 between the first and second members 92 and 94, which faces the channel 48, and the parting portion 108 that faces the optical system. Therefore, a passage that allows an inward water leakage through the parting portion 106 that faces the channel 48 is so long that the watertight function is enhanced.

Since the first and second members 92 and 94 are block-shaped, moreover, the tip forming member 31 can be parted into them even if it is made too much thinner to ensure spatial allowance.

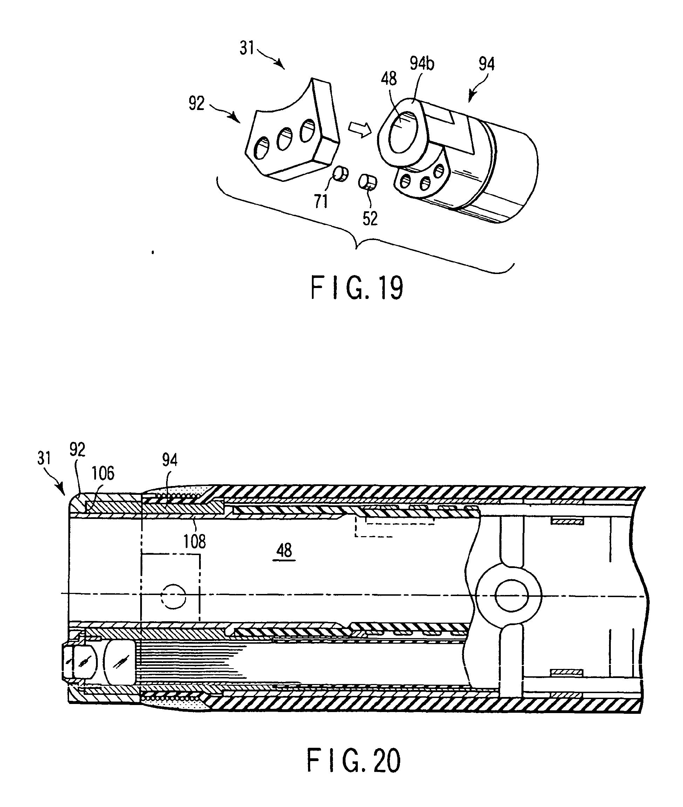

FIG. 19 shows a first modification of the fourth comparative example. Like reference numerals are used to designate like portions of this modification that have the same functions, and a description of those portions is omitted. A second member 94 of this modification has a prominence 94b on the side of a channel 48. A first member 92 has no channel-side portion and is located on the optical system side. It is in the form of a plate that can be fitted on the second member 94, as indicated by the arrow in FIG. 19. The respective opposite surfaces of the first member 92 and the prominence of the second member 94 cross a cross section that is perpendicular to the longitudinal axis of the tip forming member 31, and are located between the channel 48 and an optical system that includes a first objective lens 52 and the like.

FIG. 20 shows a fifth comparative example. Like reference numerals are used to designate like portions that have the same functions, and a description of those portions is omitted. In the present example, a cylindrical channel socket 108 that forms the outer peripheral surface of a channel 48 is fitted and fixed in a tip forming member 31. The channel socket 108 is located overlapping a parting portion 106 between first and second members 92 and 94. Thus, the parting portion 106 between the members 92 and 94 and its periphery are covered by the socket 108. Further, the channel socket 108 extends beyond the respective rear end portions of the members 92 and 94. Thus, an adequate distance is kept between the rear end portion of the socket 108 and the parting portion 106 between the members 92 and 94.

The above configuration produces the following effect. A passage that allows a water leakage from the channel 48 to the parting portion 106 is so long that the watertight function is enhanced.

## Claims

1. An endoscope (10) which has an elongate insertion section (12) to be inserted into a body cavity with a distal end portion thereof forward, the endoscope (10) further comprising:
a tip forming member (31) provided on the distal end portion and having a distal end face (33) and a housing bore (44, 51a) extending from a rear end side to a distal end side;
a protrusion (58, 75) on an inner peripheral surface of the housing bore (44, 51a);
a window (49, 50) being an illumination window or an observation window defined by the housing bore (44, 51a) and the protrusion (58, 75) within the region of the distal end face (33); and
an optical member (52, 71) which is inserted into the housing bore (44, 51a) from the rear end side and fixed to the tip forming member (31) with the distal end portion thereof run against the protrusion (58, 75);
**characterized in that** the protrusion (58, 75) is located on a distal end portion of the inner peripheral surface of the housing bore (44, 51a) and has a taper (58a, 75a) formed on the distal end side surface of the protrusion (58, 75) inclined to the distal end face (33) and smoothly connected to the distal end face (33), wherein the taper (58a, 75a) of the protrusion (58, 75) engages a distal end face of the optical member (52, 71), inclines radially from center of the window (49, 50) and spreads from the window (49, 50) towards the distal end side.

2. An endoscope according to claim 1, **characterized in that** the protrusion (58, 75) engages an edge region of the distal end face of the optical member (52, 71).

3. An endoscope (10) according to claim 1, **characterized in that** the optical member (52, 71) includes an illumination optical member (71) for illumination.

4. An endoscope (10) according to claim 1, **characterized in that** the optical member (52, 71) includes an observation optical member (52) for observation.

5. An endoscope (10) according to claim 1, **characterized in that** the endoscope (10) further comprises an additional optical member (56, 57) which cooperates with the optical member (52, 71), and the inner peripheral surface of the housing bore (44, 51a) has a distal-end-side region in which the protrusion (58, 75) and the optical member (52, 71) are arranged and a rear-end-side region which is larger in diameter than the distal-end-side region and in which at least a part of the additional optical member (56, 57) is located, and the tip forming member (31) has a stepped portion (74) which is formed between the distal- and rear-end-side regions and against which a distal end portion of the additional optical member (56, 57) is run.

6. An endoscope (10) according to claim 5, **characterized in that** the optical member (52, 71) includes an illumination lens (71) for illumination, and the additional optical member (56, 57) includes a light guide fiber (72) which supplies illumination light to the illumination lens (71).

7. An endoscope (10) according to claim 1, **characterized in that** the tip forming member (31) includes a first member (92) on the distal end side having the protrusion (58, 75) and a second member (94) on the rear end side which holds the optical member (52, 71) in cooperation with the first member (92) between itself and the first member (92).

8. An endoscope (10) according to claim 7, **characterized in that** the first member (92) is formed of a material capable of fill-curing.

9. An endoscope (10) according to claim 7, **characterized in that** the tip forming member (31) has a longitudinal axis extending in a longitudinal direction of the insertion section (12), and the first member (92) and the second member (94) are block-shaped mating members individually having parting faces across a plane perpendicular to the longitudinal axis.

10. An endoscope (10) according to claim 9, **characterized in that** the tip forming member (31) is formed having a channel (48) extending parallel to the optical member (52, 71), and the parting faces across the plane perpendicular to the longitudinal axis are located between the optical member (52, 71) and the channel (48).

11. An endoscope (10) according to claim 1, **characterized in that** the tip forming member (31) includes a mounting member (51) attached to the tip forming member (31) and having the housing bore (44, 51a).

12. An endoscope (10) according to claim 11, **characterized in that** the tip forming member (31) has an inner peripheral surface defining a through hole (43) extending from the rear end side to the distal end side, an inner peripheral surface of the through hole (43) has a first distal-end-side region and a first rear-end-side region (63) larger in diameter than the distal-end-side region, the tip forming member (31) has a first stepped portion (64) formed between the first distal-end-side region and the first rear-end-side region (63), and an outer peripheral surface of the mounting member (51) has a second distal-end-side region (61), a second rear-end-side region (60) larger in diameter than the second distal-end-side region (61), and a second stepped portion (62) formed between the second distal-end-side region (61) and the second rear-end-side region (60) and running against the first stepped portion (64).

13. An endoscope (10) according to claim 11, **characterized in that** the optical member (52, 71) includes an objective lens (52) for observation of which a distal end portion is run against the protrusion (58, 75), and the mounting member (51) includes a lens frame (51) having the housing bore (44, 51a).

14. An endoscope (10) according to claim 11, **characterized in that** the tip forming member (31) includes a first member (92) on the distal end side having the protrusion (58, 75) and a second member (94) on the rear end side which holds the mounting member (51) in cooperation with the first member (92) between itself and the first member (92).

15. An assembly method for an endoscope (10), which has an elongate insertion section (12) to be inserted into a body cavity with a distal end portion thereof forward, the method comprising:
inserting an optical member (52, 71) into a housing bore (44, 51a), which penetrates a tip forming member (31) on the distal end portion from a rear end side to a distal end side, from the rear end side;
positioning the optical member (52, 71) at a distal end face region of the tip forming member (31) by running a distal end portion of the optical member (52, 71) against a protrusion (58, 75) on an inner peripheral surface of the housing bore (44, 51a), wherein a window (49, 50) being an illumination window or an observation window is defined by the housing bore (44, 51a) and the protrusion (58, 75) within the distal end face region, and a taper (58, 75a) formed on the distal end side surface of the protrusion (58, 75) is inclined to the distal end face (33) of the tip forming member (31) and smoothly connected to the distal end face and engages a distal end face of the optical member (52, 71), inclines radially from the center of the window (49, 50) and spreads from the window (49, 50) towards the distal end side; and
fixing the optical member (52, 71) to the tip forming member (31).

16. An assembly method for an endoscope (10) according to claim 15, **characterized in that** the positioning includes positioning the optical member (52, 71) by running the distal end portion of the optical member (52, 71) against the protrusion (58, 75), in a distal-end-side region (61) of the inner peripheral surface of the housing bore (44, 51a) in which the protrusion (58, 75) is located, and positioning an additional optical member (56, 57), which cooperates with the optical member (52, 71), by running a distal end portion of the additional optical member (56, 57) against a stepped portion (74), which is formed between the distal-end-side region (61) and a rear-end-side region (60) larger in diameter than the distal-end-side region (61), in the rear-end-side region (60) of the inner peripheral surface of the housing bore (44, 51a).

17. An assembly method for an endoscope (10) according to claim 15, **characterized in that** the fixing includes holding the optical member (52, 71), by means of a first member (92) on the distal end side of the tip forming member (31) which has the protrusion (58, 75) and a second member (94) on the rear end side of the tip forming member (31), between the first member (92) and the second member (94).

## Patentansprüche

1. Ein Endoskop (10), welches einen lang gestreckten Einführabschnitt (12) zum Einführen in eine Körperhöhle mit einem nach vorne gerichteten distalen Endabschnitt aufweist, wobei das Endoskop (10) weiterhin aufweist:
ein Spitzenbildungsteil (31), das an dem distalen Endabschnitt angeordnet ist und eine distale Endfläche (33) und eine Aufnahmebohrung (54, 51a) hat, die sich von einer hinteren Endseite zu einer distalen Endseite erstreckt;
einen Vorsprung (58, 75) an einer inneren Umfangsoberfläche der Aufnahmebohrung (44, 51a);
ein Fenster (49, 50), welches ein Beleuchtungsfenster oder ein Beobachtungsfenster ist und durch die Aufnahmebohrung (44, 51a) und den Vorsprung (58, 75) innerhalb des Bereichs der distalen Endfläche (33) definiert ist; und
ein optisches Bauteil (52, 71), das in die Aufnahmebohrung (44, 51a) von der hinteren Endseite her eingefügt und an dem Spitzenbildungsteil (31) befestigt ist, wobei der distale Endabschnitt hiervon an dem Vorsprung 858, 75) aufläuft;
**dadurch gekennzeichnet, dass** der Vorsprung (58, 75) an einem distalen Endabschnitt der inneren Umfangsoberfläche der Aufnahmebohrung (44, 51a) liegt und eine Abschrägung (58a, 75a) hat, die an der distalen Endseitenfläche des Vorsprungs (58, 75) ausgebildet, zur distalen Endfläche (33) hin geneigt und mit der distalen Endfläche (33) mit einem glatten Übergang verbunden ist, wobei die Abschrägung (58a, 75a) des Vorsprungs (58, 75) in Anlage mit einer distalen Endfläche des optischen Bauteils (52, 71) ist, radial von der Mitte des Fensters (49, 50) aus geneigt ist und von dem Fenster (49, 50) in Richtung der distalen Endseite verläuft.

2. Ein Endoskop nach Anspruch 1, **dadurch gekennzeichnet, dass** der Vorsprung (58, 75) mit einem Kantenbereich der distalen Endfläche des optischen Bauteils (52, 71) in Anlage ist.

3. Ein Endoskop (10) nach Anspruch 1, **dadurch gekennzeichnet, dass** das optische Bauteil (52, 71) ein optisches Beleuchtungsteil (71) für Beleuchtung enthält.

4. Ein Endoskop (10) nach Anspruch 1, **dadurch gekennzeichnet, dass** das optische Bauteil (52, 71) ein optisches Beobachtungsbauteil (52) für die Beobachtung enthält.

5. Ein Endoskop (10) nach Anspruch 1, **dadurch gekennzeichnet, dass** das Endoskop ein zusätzliches optisches Bauteil aufweist, das mit dem optischen Bauteil (52, 71) zusammenwirkt, wobei die innere Umfangsoberfläche der Aufnahmebohrung der (44, 51a) einen distal endseitigen Bereich, in welchem der Vorsprung (58, 75) und das optische Bauteil (52, 71) angeordnet sind und einen hinterendseitigen Bereich hat, der größeren Durchmesser als der distal endseitige Bereich hat und in welchem zumindest ein Teil des zusätzlichen optischen Bauteils (56, 57) liegt, wobei das Spitzenbildungsteil (31) einen abgestuften Abschnitt (74) hat, der zwischen den distal endseitigen und hinterendseitigen Bereichen ausgebildet ist und an dem ein distaler Endabschnitt des zusätzlichen optischen Bauteils (56,57) aufläuft.

6. Ein Endoskop (10) nach Anspruch 5, **dadurch gekennzeichnet, dass** das optische Bauteil (52, 71) eine Beleuchtungslinse (71) für Beleuchtung aufweist und dass das zusätzliche optische Bauteil (56, 57) eine Lichtleitfaser (72) enthält, welche Beleuchtungslicht zu der Beleuchtungslinse (71) liefert.

7. Ein Endoskop (10) nach Anspruch 1, **dadurch gekennzeichnet, dass** das Spitzenbildungsteil (31) ein erstes Bauteil (92) an der distalen Endseite mit dem Vorspruch (58, 75) und ein zweites Bauteil an der Hinterendseite enthält, welches das optische Bauteil (52, 71) in Zusammenwirkung mit dem ersten Bauteil (92) zwischen sich und dem ersten Bauteil (92) hält.

8. Ein Endoskop (10) nach Anspruch 7, **dadurch gekennzeichnet, dass** das erste Bauteil (92) aus einem Material gebildet ist, welches einfüllbar/aushärtend ist.

9. Ein Endoskop (10) nach Anspruch 7, **dadurch gekennzeichnet, dass** das Spitzenbauteil eine Längsachse hat, die sich in einer Längsrichtung des Einführabschnitts (12) erstreckt, wobei das erste Bauteil (92) und das zweite Bauteil (94) blockförmige zusammenpassende Teile sind, welche jeweils Teilungsflächen über ein Ebene senkrecht zur Längsachse haben.

10. Ein Endoskop (10) nach Anspruch 9, **dadurch gekennzeichnet, dass** das Spitzenbildungsteil (31) so gebildet ist, dass einen Kanal (48) bildet, der sich parallel zum optischen Bauteil (52, 71) erstreckt, wobei die Teilungsflächen über die Ebene senkrecht zur Längsachse zwischen dem optischen Bauteil (52, 71) und dem Kanal (48) liegen.

11. Ein Endoskop (10) nach Anspruch 1, **dadurch gekennzeichnet, dass** das Spitzenbildungsteil (31) ein Aufnahmeteil (51) enthält, das an dem Spitzenbildungsteil (31) angebracht ist und die Aufnahmebohrung (44, 51a) enthält.

12. Ein Endoskop (10) nach Anspruch 11, **dadurch gekennzeichnet, dass** das Spitzenbildungsteil (31) eine innere Umfangsfläche hat, die eine Durchgangsbohrung (43) definiert, welche sich von der hinteren Endseite zur distalen Endseite erstreckt, wobei eine innere Umfangsoberfläche der Durchgangsbohrung (43) einen ersten distal endseitigen Bereich und einen ersten hinterendseitigen Bereich (63) mit größerem Durchmesser wie der distal endseitige Bereich hat, das Spitzenbildungsteil (31) einen ersten abgestuften Abschnitt (64) zwischen dem ersten distal endseitigen Bereich und dem ersten hinterendseitigen Bereich (63) hat und eine äußere Umfangsoberfläche des Halteteils (51) einen zweiten distal endseitigen Bereich (61), einen zweiten hinterendseitigen Bereich (60) mit größerem Durchmesser wie der zweite distal endseitige Bereich (61) und einen zweiten abgestuften Abschnitt (62) hat, der zwischen dem zweiten distalen endseitigen Bereich (61) und dem zweiten hinterendseitigen Bereich ausgebildet ist und an dem ersten abgestuften Abschnitt (64) aufläuft.

13. Ein Endoskop (10) nach Anspruch 11, **dadurch gekennzeichnet, dass** das optische Bauteil (52, 71) eine Objektivlinse (2) zur Beobachtung enthält, von der ein distaler Endabschnitt an dem Vorsprung (58, 75) aufläuft, wobei das Aufnahmeteil (51) einen Linsenrahmen (51) mit der Aufnahmebohrung (44, 51a) enthält.

14. Ein Endoskop (10) nach Anspruch 11, **dadurch gekennzeichnet, dass** das Spitzungsbildungsteil (31) ein erstes Bauteil (92) an der distalen Endseite mit dem Vorsprung (58, 75) und ein zweites Bauteil (94) an der hinteren Endseite enthält, welches das Halteteil (51) zusammen mit dem ersten Bauteil (92) zwischen sich und dem ersten Bauteil (92) hält.

15. Ein Zusammenbauverfahren für ein Endoskop (10), welches einen lang gestreckten Einführabschnitt (12) zum Einführen in eine Körperhöhle mit einem nach vorne gerichteten distalen Endabschnitt hat, wobei das Verfahren aufweist:
Einführen eines optischen Bauteils (52, 71) in eine Aufnahmebohrung (44, 51a), welche ein Spitzenbildungsteil (31) am distalen Endabschnitt von einer hinteren Endseite zu einer distalen Endseite durchtritt, von der hinteren Endseite her;
Positionieren eines optischen Bauteils 52, 71) an einem distalen Endflächenbereich des Spitzenbildungsteils (31) durch Auflaufenlassen eines distalen Endabschnitts des optischen Bauteils (52, 71) an einem Vorsprung (58, 75) an einer inneren Umfangsoberfläche der Aufnahmebohrung (44, 51a), wobei ein Fenster (49, 50), das ein Beleuchtungsfenster oder ein Beobachtungsfenster ist, durch die Aufnahmebohrung (44, 51a) und den Vorsprung (58, 75) innerhalb des distalen Endflächenbereichs definiert ist und eine Abschrägung (58, 75a), die an der distal endseitigen Fläche des Vorsprungs (58, 75) ausgebildet ist, zu der distalen Endfläche (33) des Spitzenbildungsteils (31) geneigt ist und mit glattem Übergang mit der distalen Endfläche verbunden und in Anlage mit einer distalen Endfläche des optischen Bauteils (52, 71) ist, radial von der Mitte des Fensters (49, 50) aus geneigt ist und sich von dem Fenster (49, 50) in Richtung der distalen Endseite erstreckt; und
Befestigen des optischen Bauteils (52, 71) an dem Spitzenbildungsteil (31).

16. Ein Zusammenbauverfahren für ein Endoskop (10) nach Anspruch 15, **dadurch gekennzeichnet, dass** das Positionieren des optischen Bauteils (52, 71) das Auflaufenlassen des distalen Endabschnitts des optischen Bauteils (52, 71) an dem Vorsprung (58, 75) in einem distal endseitigen Bereich (61) der inneren Umfangsoberfläche der Aufnahmebohrung (44, 51) in der der Vorsprung (58, 75) liegt, aufweist, sowie das Positionieren eines zusätzlichen optischen Bauteils (56, 57), das mit dem optischen Bauteil (52, 71) zusammen wirkt, durch Auflaufenlassen eines distalen Endabschnitts eines zusätzlichen optischen Bauteils (56, 57) an einem abgestuften Abschnitt (74), der zwischen dem distal endseitigen Bereich (61) und einem hinterendseitigen Bereich (60) mit größerem Durchmesser wie der distal endseitige Bereich (61) ausgebildet ist, in dem hinterendseitigen Bereich (60) der inneren Umfangsoberfläche der Aufnahmebohrung (44, 51a).

17. Ein Zusammenbauverfahren für ein Endoskop (10) nach Anspruch 15, **dadurch gekennzeichnet, dass** das Befestigen das Halten des optischen Bauteils (52, 71) mittels eines ersten Bauteils (92) und der distalen Endseite des Spitzenbildungsteils (31), das den Vorsprung (58, 75) hat und eines zweiten Bauteils (94) an der hinteren Endseite des Spitzenbildungsteils (31) zwischen dem ersten Bauteil (92) und dem zweiten Bauteil (94) enthält.

## Revendications

1. Endoscope (10) qui comporte une section d'insertion allongée (12) à insérer dans une cavité corporelle avec une partie d'extrémité distale de celle-ci vers l'avant, l'endoscope (10) comprenant en outre :
un élément formant un embout (31) pourvu sur la partie d'extrémité distale et comportant une face d'extrémité distale (33) et un alésage (44, 51a) s'étendant d'un côté de l'extrémité arrière à un côté de l'extrémité distale ;
une saillie (58, 75) sur une surface périphérique interne de l'alésage (44, 51a) ;
une fenêtre (49, 50) étant une fenêtre d'éclairage ou une fenêtre d'observation définie par l'alésage (44, 51a) et la saillie (58, 75) à l'intérieur de la région de la face d'extrémité distale (33) ; et
un élément optique (52, 71) qui est inséré dans l'alésage (44, 51a) depuis le côté de l'extrémité arrière et fixé à l'élément formant un embout (31) avec la partie d'extrémité distale de celui-ci amenée contre la saillie (58, 75) ;
**caractérisé en ce que** la saillie (58, 75) est située sur une partie d'extrémité distale de la surface périphérique interne de l'alésage (44, 51a) et comporte un biseau (58a, 75a) formé sur la surface du côté de l'extrémité distale de la saillie (58, 75) incliné vers la face d'extrémité distale (33) et relié de façon lisse à la face d'extrémité distale (33), dans lequel le biseau (58a, 75a) de la saillie (58, 75) s'engage avec une face d'extrémité distale de l'élément optique (52, 71), s'incline radialement depuis le centre de la fenêtre (49, 50) et s'étend depuis la fenêtre (49, 50) vers le côté de l'extrémité distale.

2. Endoscope selon la revendication 1, **caractérisé en ce que** la saillie (58, 75) s'engage avec une région de bord de la face d'extrémité distale de l'élément optique (52, 71).

3. Endoscope (10) selon la revendication 1, **caractérisé en ce que** l'élément optique (52, 71) comprend un élément optique d'éclairage (71) pour éclairage.

4. Endoscope (10) selon la revendication 1, **caractérisé en ce que** l'élément optique (52, 71) comprend un élément optique d'observation (71) pour l'observation.

5. Endoscope (10) selon la revendication 1, **caractérisé en ce que** l'endoscope (10) comprend en outre un élément optique supplémentaire (56, 57) qui coopère avec l'élément optique (52, 71), et la surface périphérique interne de l'alésage (44, 51a) comporte une région de côté d'extrémité distale dans laquelle la saillie (58, 75) et l'élément optique (52, 71) sont disposés et une région de côté d'extrémité arrière qui est plus grande en diamètre que la région de côté d'extrémité distale et dans laquelle au moins une partie de l'élément optique supplémentaire (56, 57) est située, et l'élément formant un embout (31) comporte une partie en gradins (74) qui est formée entre les régions côté extrémité distale et arrière et contre laquelle une partie d'extrémité distale de l'élément optique supplémentaire (56, 57) est amenée.

6. Endoscope (10) selon la revendication 5, **caractérisé en ce que** l'élément optique (52, 71) comprend une lentille d'éclairage (71) pour l'éclairage, et l'élément optique supplémentaire (56, 57) comprend une fibre de guide de la lumière (72) qui fournit une lumière d'éclairage à la lentille d'éclairage (71).

7. Endoscope (10) selon la revendication 1, **caractérisé en ce que** l'élément formant un embout (31) comprend un premier élément (92) sur le côté de l'extrémité distale ayant la saillie (58, 75) et un deuxième élément (94) sur le côté de l'extrémité arrière qui maintient l'élément optique (52, 71) en coopération avec le premier élément (92) entre lui-même et le premier élément (92).

8. Endoscope (10) selon la revendication 7, **caractérisé en ce que** le premier élément (92) est réalisé dans un matériau susceptible de durcir par remplissage.

9. Endoscope (10) selon la revendication 7, **caractérisé en ce que** l'élément formant un embout (31) comporte un axe longitudinal s'étendant dans une direction longitudinale de la section d'insertion (12), et le premier élément (92) et le deuxième élément (94) sont des éléments conjugués en forme de blocs ayant individuellement des faces de joint à travers un plan perpendiculaire à l'axe longitudinal.

10. Endoscope (10) selon la revendication 9, **caractérisé en ce que** l'élément formant un embout (31) est formé en ayant un canal (48) s'étendant parallèlement à l'élément optique (52, 71), et les faces de séparation à travers le plan perpendiculaire à l'axe longitudinal sont situées entre l'élément optique (52, 71) et le canal (48).

11. Endoscope (10) selon la revendication 1, **caractérisé en ce que** l'élément formant un embout (31) comprend un élément de montage (51) attaché à l'élément formant un embout (31) et comportant l'alésage (44, 51a).

12. Endoscope (10) selon la revendication 11, **caractérisé en ce que** l'élément formant un embout (31) comporte une surface périphérique interne définissant un trou traversant (43) s'étendant du côté de l'extrémité arrière au côté de l'extrémité distale, une surface périphérique interne du trou traversant (43) comporte une première région de côté d'extrémité distale et une première région de côté d'extrémité arrière (63) plus grande en diamètre que la région de côté d'extrémité distale, l'élément formant un embout (31) comporte une première partie en gradins (64) formée entre la première région de côté d'extrémité distale et la première région de côté d'extrémité arrière (63), et une surface périphérique externe de l'élément de montage (51) comporte une deuxième région de côté d'extrémité distale (61), une deuxième région de côté d'extrémité arrière (60) plus grande en diamètre que la deuxième région de côté d'extrémité distale (61), et une deuxième partie en gradins (62) formée entre la deuxième région de côté d'extrémité distale (61) et la deuxième région de côté d'extrémité arrière (60) et arrivant contre la première partie en gradins (64).

13. Endoscope (10) selon la revendication 11, **caractérisé en ce que** l'élément optique (52, 71) comprend un objectif (52) pour l'observation dont une partie d'extrémité distale est amenée contre la saillie (58, 75), et l'élément de montage (51) comprend une monture de lentille (51) comportant l'alésage (44, 51a).

14. Endoscope (10) selon la revendication 11, **caractérisé en ce que** l'élément formant un embout (31) comprend un premier élément (92) sur le côté de l'extrémité distale ayant la saillie (58, 75) et un deuxième élément (94) sur le côté de l'extrémité arrière qui maintient l'élément de montage (51) en coopération avec le premier élément (92) entre lui-même et le premier élément (92).

15. Procédé d'assemblage pour un endoscope (10) qui comporte une section d'insertion allongée (12) à insérer dans une cavité corporelle avec une partie d'extrémité distale de celle-ci vers l'avant, le procédé comprenant :
l'insertion d'un élément optique (52, 71) dans un alésage (44, 51a) qui pénètre dans un élément formant un embout (31) sur la partie d'extrémité distale du côté de l'extrémité arrière au côté de l'extrémité distale, depuis le côté de l'extrémité arrière ;
le positionnement de l'élément optique (52, 71)) dans une région de la face d'extrémité distale de l'élément formant un embout (31) en amenant une partie d'extrémité distale de l'élément optique (52, 71) contre une saillie (58, 75) sur une surface périphérique interne de l'alésage (44, 51a), dans lequel une fenêtre (49, 50) étant une fenêtre d'éclairage ou une fenêtre d'observation est définie par l'alésage (44, 51a) et la saillie (58, 75) à l'intérieur de la région de la face d'extrémité distale, et un biseau (58, 75a) formé sur la surface du côté de l'extrémité distale de la saillie (58, 75) est incliné vers la face d'extrémité distale (33) de l'élément formant un embout (31) et relié de façon lisse à la face d'extrémité distale et s'engage avec une face d'extrémité distale de l'élément optique (52, 71), s'incline radialement depuis le centre de la fenêtre (49, 50) et s'étend depuis la fenêtre (49, 50) vers le côté de l'extrémité distale ; et
la fixation de l'élément optique (52, 71) à l'élément formant un embout (31).

16. Procédé d'assemblage pour un endoscope (10) selon la revendication 15, **caractérisé en ce que** le positionnement comprend le positionnement de l'élément optique (52, 71) en amenant la partie d'extrémité distale de l'élément optique (52, 71) contre la saillie (58, 75), dans une région de côté d'extrémité distale (61) de la surface périphérique interne de l'alésage (44, 51a) dans laquelle la saillie (58, 75) est située, et le positionnement d'un élément optique supplémentaire (56, 57), qui coopère avec l'élément optique (52, 71), en amenant une partie d'extrémité distale de l'élément optique supplémentaire (56, 57) contre une partie en gradins (74), qui est formée entre la région côté extrémité distale (61) et une région de côté d'extrémité arrière (60) plus grande en diamètre que la région de côté d'extrémité distale (61), dans la région de côté d'extrémité arrière (60) de la surface périphérique interne de l'alésage (44, 51a).

17. Procédé d'assemblage pour un endoscope (10) selon la revendication 15, **caractérisé en ce que** la fixation comprend le maintien de l'élément optique (52, 71), au moyen d'un premier élément (92) sur le côté de l'extrémité distale de l'élément formant un embout (31) qui a la saillie (58, 75) et d'un deuxième élément (94) sur le côté de l'extrémité arrière de l'élément formant un embout (31), entre le premier élément (92) et le deuxième élément (94).
